# EUROPÄISCHE PATENTSCHRIFT

(11) **EP 0 910 624 B1**
(45) Veröffentlichungstag und Bekanntmachung des Hinweises auf die Patenterteilung: **22.09.2004**
(21) Anmeldenummer: 97919378.6
(22) Anmeldetag: 17.04.1997
(51) Int. Cl.: C12N 5/08

(54) **VERFAHREN ZUR HERSTELLUNG UND VERMEHRUNG VON LYMPHOZYTEN**
PROCESS FOR PRODUCING AND MULTIPLYING LYMPHOCYTES
PROCEDE DE PRODUCTION ET DE MULTIPLICATION DE LYMPHOCYTES

(30) Priorität: 17.04.1996 EP 96105993
(43) Veröffentlichungstag der Anmeldung: 28.04.1999
(73) Patentinhaber: Albert, Winfried, Dr., 82362 Weilheim (DE)
(72) Erfinder: JUNGFER, Herbert, D-82319 Starnberg (DE); BARCHET, Heinrich, D-82347 Bernried (DE); ALBERT, Winfried, D-82390 Eberfing (DE)
(74) Vertreter: Büchel, Kurt F.
(86) Internationale Anmeldenummer: PCT/EP1997/001924
(87) Internationale Veröffentlichungsnummer: WO 1997/039108

(56) Entgegenhaltungen:
- EP-A- 0 409 655
- BLOOD, Bd. 87, Nr. 1, Januar 1996, Seiten 180-189, XP000604748 PIERSON B.A. ET AL: "Human natural killer cell expansion is regulated by thrombospondin-mediated activation of transforming growth factor-beta1 and independent accesory cell-derived contact and soluble factors"
- PERIODICUM BIOLOGORUM, Bd. 92, Nr. supp3, 1990, Seite 48 XP002014509 KOTNIK V. ET AL: "MRL reactivity renewal of mouse lymphocytes in vitro treated with ciclosporin and human rIL-2"
- IMMUNOPHARMACOLOGY AND IMMUNOTOXICOLOGY, Bd. 16, Nr. 2, 1994, Seiten 179-190, XP002035237 WEAVER, J.L. ET AL: "The interaction of immunosuppressive compounds in tandem stimulated peripheral human lymphocytes"

## Beschreibung

Gegenstand der Erfindung ist ein Verfahren zur Herstellung und Vermehrung von Lymphozyten sowie eine Zusammensetzung, welche sich als Kulturmedium für Lymphozyten eignet.

Die Herstellung und Vermehrung von Lymphozyten ist problematisch. Manche Lymphozyten-Arten sind in vitro überhaupt nicht oder sehr schwierig kultivierbar. Native B-Lymphozyten sind beispielsweise nur kurzzeitig kultivierbar, und T-Lymphozyten erfordern zur längeren Kultivierung diffizile Kulturbedingungen, wie die Kombination von Wachstumsfaktor(en) und "feeder cells" ("Ammen-Zellen") oder den Einsatz von unphysiologischen und potentiell gefährlichen Substanzen wie Tumorpromotoren (Phorbolester) in Kombination mit ionophoren Substanzen (z. B. Ionomycin) oder pflanzlichen Lektinen (z. B. Phythämagglutin, PHA). Dadurch sind die Möglichkeiten zur Langzeitkultivierung und Vermehrung von T- und anderen Lymphozyten sehr begrenzt, und die Herstellung von Lymphozyten in nennenswerten Mengen zu diagnostischen oder therapeutischen Zwecken ist stark eingeschränkt oder unmöglich.

Dabei besteht ein vielfältiges Interesse, Lymphozyten in vitro herstellen, kultivieren und vermehren zu können, beispielsweise B-Lymphozyten als Produzenten von spezifischen Antikörpern, oder zytotoxische T-Lymphozyten (CTL) zur Behandlung von Infektionen oder Tumoren, weiterhin Regulator-T-Lymphozyten (Helfer- bzw. Suppressor-T-Lymphozyten) zur Diagnose und Behandlung von Autoimmunkrankheiten, oder "Natural-Killer (NK)"-Lymphozyten für die Bekämpfung von Malignomen.

Ohne Schwierigkeiten kultivierbar und vermehrbar sind Hybridomzellen, die aus einer Fusion von B-Lymphozyten oder von T-Lymphozyten mit malignen, lymphoiden Zellen (z. B. Myelomzellen) entstehen. Solche Hybridomzellen besitzen sowohl die immunologische Funktion der Ausgangslymphozyten als auch die im wesentlichen unlimitierte Proliferationsfähigkeit der malignen Fusionspartner. Die Anwendung der Hybridomatechnik ist aber auf wenige Tierspezies (Maus, Ratte) beschränkt und versagt im wesentlichen bei anderen Saugerarten, insbesondere auch beim Menschen.

In der WO90/10059 wird ein Verfahren zur Herstellung von proliferierenden CD4+-Lymphozyten beschrieben. Danach werden periphere mononukleäre Blutzellen (PBNMC) zur Entfernung von Monozyten und Granulozyten mit Alkylestern behandelt und anschließend in einem Kulturmedium kultiviert, welches ein T-Zell-Stimulans und/oder IL-2 enthält. Als T-Zell-Stimulans werden Mitogene, wie PHA verwendet. Die Verwendung von IL-2 allein führt jedoch nur zu einer geringen Vermehrung der Zellen. Der Zusatz von mitogenen Substanzen bei der Kultivierung von Zellen, die anschließend dem Patienten implantiert werden sollen, ist kritisch.

In der EP-A 0 203 403 ist ebenfalls ein Verfahren zur Kultivierung von T-Zellen, in Gegenwart von Interleukin-2, beschrieben. Auch hier besteht der Nachteil des Verfahrens darin, daß die T-Zellen dadurch nur in geringem Umfang vermehrt werden können.

In der WO94/23014 wird ein Verfahren zur Kultivierung und Vermehrung von tumoriziden T-Lymphozyten beschrieben, durch Kokultivierung von Lymphozyten mit einer Zellinie (Stimulatorzellen), unter Vermeidung einer allogenen Stimulierung und ohne Zusatz von Interleukin-2. Dabei werden ruhende T-Lymphozyten zu Effektorzellen aktiviert, die Tumorzellen erkennen und abtöten oder im Wachstum inhibieren. Die Bereitstellung von solchen Stimulatorzellen für die Massenvermehrung von beispielsweise tumoriziden Killer-T-Zellen, erfordert einen erheblichen Fermentieraufwand. Außerdem müssen die Killer-T-Zellen vor Verwendung (Reinfusion in den Patienten) von diesen Stimulatorzellen bzw. deren Zelltrümmer steril abgetrennt werden.

Von V. Kutnik et al., Period. Biol. 92 (1990) 48 wird beschrieben daß in der allogenen "mixed lymphocyte reaction" von Maus-Milz-Lymphozyten die Zugabe von IL2, die durch Cyclosporin A induzierte Hemmung der Proliferation der responder-Zellen restauriert und deren Alloreaktivität steigert.
Von Pierson, B.A. et al., Blood 87 (1996) 180 - 189 wird beschrieben, daß isolierte humane NK-Zellen (CD56+, CD3-) in Kultur durch Zugabe von IL2 geringfügig vermehrt werden können. Die Zugabe von Überständen bestrahlter mononukleärer Zellen aus Blut verstärkt die Vermehrung der NK-Zellen. Als wirksames Prinzip dieses Effekts wird Thrombospondin identifiziert, das nicht direkt sondern indirekt durch Aktivierung von latentem TGFβ wirkt. So aktiviertes TGFβ hemmt die Proliferation in der frühen Phase der Kultur und steigert die Proliferation im weiteren. Ein wiederholter Zusatz von TGFβ bei Mediumwechsel inhibiert das Auswachsen der Zellen und unterdrückt hier die Proliferation der NK-Zellen.

Von I.A. Ayoub wird in Immunological Invest. 25 (1996) 129 - 151 die Auswirkung von humanem TGFβ auf eine Bovine CD4+ lymphoblastoide T-Zellinie (BLTC) untersucht, die in IL2-haltigem Medium autonom wächst. Im Medium ohne IL2 wird die Zellinie arretiert. Zugabe von TGFβ zu den arretierten BLTC treibt diese beschleunigt in Apoptose. Gleichzeitige Addition von IL2 hebt die Arretierung auf und verhindert die Induktion von Apoptose. Die Zugabe von TGFβ zu suboptimalen Konzentrationen von IL2 costimuliert die Proliferation der BLTC.

R. de Jong beschreibt in Intern. Immunol. 6 (1994) 631 - 638 die Wirkung von TGFβ1 auf die Proliferation von isolierten Subpopulationen von humanem CD4+ T-Lymphozyten. TGFβ1 verstärkt die durch Antikörper induzierte Proliferation von CD4-Zellen (CD45 RA+) in Gegenwart von IL2, die Proliferation von voraktivierten T-Zellen (CD45 RO+) wird dagegen durch die Zugabe von TGFβ1 gehemmt. Dabei zeigt sich allerdings, daß die induzierte Proliferation der CD45 RA+-Zellen nach fünf Tagen durch Zugabe von TGFβ1 gehemmt wird.

Von A. Cerwenka wird in J. Immunol. 156 (1996) 459 - 464 beschrieben, daß die Anwesenheit von TGFβ1 bei der primären Stimulation von humanen T-Lymphozyten, deren Überlebensfähigkeit in sekundären Kulturen erhöht, ihre Anfälligkeit gegenüber Apoptose induzierenden Anti-Fas-Antikörpern vermindert. Ebenso vermindert die Zugabe von TGFβ1 die Apoptoseanfälligkeit der primär aktivierten T-Lymphozyten gegenüber sekundärer Aktivierung. In Gegenwart von IL2 und TGFβ1 wird ein Überleben der T-Zellen über lange Zeit gewährleistet.

Von T.H. Inne et al., J. Immunol. 148 (1992) 3847 - 3856 wird beschrieben, daß die Proliferation von CTLL-2-Zellen (murine T-Zellinie) in IL2-haltigen Medien durch Zugabe von TGF β1 gehemmt wird. Neben der Proliferationshemmung, induziert TGFβ1 in CTLL-2 eine Änderung der Zellmorphologie und induziert die Expression des Oberflächenmoleküls CD8. Durch die Kombination von IL2 und TGFβ wird auch in murinen Thymozyten, die durch Phorboldibutyrat und Ionomycin aktiviert werden, eine verstärkte Expression von CD8 induziert. Die Zugabe von TGFβ1 bewirkt auch hier eine Verminderung der Proliferationsrate.

TGFβ ist allerdings keine Substanz, welche ohne weiteres in ausreichenden Mengen und kostengünstig verfügbar ist. TGFβ wird üblicherweise entweder aus natürlichen Quellen isoliert oder rekombinant hergestellt. Die Aufgabe der Erfindung liegt im wesentlichen darin, effektive und kostengünstige Mittel zur Kultivierung und Vermehrung von Lymphozyten sowie ein Verfahren zur Herstellung von pancytotoxischen T-Zellen bereitzustellen. Eine weitere Aufgabe der vorliegenden Erfindung besteht darin, ein Verfahren bereitzustellen, mit dem Lymphozyten auf einfache Weise und in großem Umfang hergestellt, kultiviert und/oder vermehrt werden können.

Gegenstand der Erfindung ist ein Verfahren zur Kultivierung und/oder Vermehrung von Lymphozyten in einem Zellkulturmedium, welches einen Lymphozyten-Wachstumsfaktor und zusätzlich Aurintricarbonsäure, Cyclosporin und/oder Ascomycin enthält.

Überraschenderweise wird mit dem erfindungsgemäßen Verfahren erreicht, daß Lymphozyten mit speziellen Eigenschaften auf einfache Weise herstellbar sind, über lange Zeit kultivierbar und in beträchtlichem Umfang vermehrbar sind. Das erfindungsgemäße Verfahren ist insbesondere zur Kultivierung und Vermehrung von T-Lymphozyten und NK-Lymphozyten geeignet.

Es hat sich weiter gezeigt, daß das erfindungsgemäße Verfahren besonders vorteilhaft die Kultivierung von Lymphozyten über einen langen Zeitraum (mehr als 14 Tage) und ihre Vermehrung in großem Umfang (Faktor 100, 1000 oder mehr) ermöglicht. Außerdem gelingt es mit einer Kombination eines Lymphozyten-Wachstumsfaktors und zusätzlich Aurintricarbonsäure, Cyclosporin und/oder Ascomycin aus Lymphozytengemischen durch Langzeitkultivierung pancytotoxische T-Zellen, bevorzugt aus einer Lymphozytenzellpopulation zu vermehren und dadurch derartige Zellen einfach und in großen Mengen herzustellen.

Unter einem Lymphozyten-Wachstumsfaktor ist eine Substanz zu verstehen, welche in der Lage ist, die Zellteilung von Lymphozyten zu fördern. Lymphozyten-Wachstumsfaktoren sind dem Fachmann im weiten Umfang bekannt. Geeignete T-Lymphozyten-Wachstumsfaktoren sind beispielsweise Interleukin-2 (IL-2) und Interleukin-15 (IL-15). Ein geeigneter NK-Lymphozyten-Wachstumsfaktor ist z. B. IL-15. Geeignete B-Lymphozyten-Wachstumsfaktoren sind beispielsweise Interleukin-13 (IL-13), IL-14 und IL-10 (Callard, R.E., und Gearing, J.H., The Cytokine Facts Book, Academic Press, London, 1994).

Erfindungsgemäß können überraschenderweise zur Proliferationssteigerung Cyclosporin (z.B. Cyclosporin A), Ascomycin (FK520) und/oder Tacrolimus/FK506) verwendet werden. Bei diesen Substanzen handelt es sich um Substanzen, die an ein Cyclophilin binden und in diesem Komplex Calcineurin inhibieren. Auch weitere Substanzen, die diese Eigenschaften besitzen, sind erfindungsgemäß geeignet. Pazderka-F., et al., Transpl-Immunol. (1996) 23 - 31, Rusnak-F., et al., Bone-Marrow-Transplant. 17 (1996) 309 - 13, Su-Q., et al., Ren-Physiol-Biochem. 18 (1995) 128 - 39, Baughman-G., et al., Mol-Cell-Biol. 15 (1995) 4395 - 402, Kawamura, A., et al., J-Biol-Chem. 270 (1995) 15463 - 6, Kakalis, LT., et al., FEBS-Lett. 362 (1995) 55 - 8.

Überraschenderweise kann auch Aurintricarbonsäure (ATA) zur Proliferationssteigerung von Lymphozyten verwendet werden. Aurintricarbonsäure ist eine Substanz, welche die Interaktion von Proteinen und Nukleinsäuren hemmt [Gonzales, R.G. et al.: Biochemistry 19: 4299 - 4303 (1980)] und ein genereller Inhibitor von Nucleasen ist.

Die Menge an Aurintricarbonsäure, die im erfindungsgemäßen Verfahren zugesetzt wird, kann ebenfalls variieren und ist im gewissen Umfang vom verwendeten Medium und von der zu kultivierenden Zelle abhängig. Es hat sich gezeigt, daß es z.B. bei der Kultivierung von Tumor-infiltrierenden Lymphozyten (TIL) in serumhaltigem Medium vorteilhaft ist, 0,1 - 100 µM ATA zuzusetzen.

Unter Lymphozyten, im Sinne der Erfindung, sind Leukozyten zu verstehen, die im hämatopoietischen System aus Lymphozyten-Progenitor-Zellen hervorgehen und z. B. im Blut, in der Lymphe, in der Milz, in Lymphknoten, in Tumoren (tumorinfiltrating lymphocytes, TIL) oder entzündeten Geweben zu finden sind. Wesentliche Untergruppen sind T-Lymphozyten, NK-Lymphozyten und B-Lymphozyten. B-Lymphozyten sind in ihrer reifen Form Antikörperproduzierende Lymphozyten.

Unter T-Lymphozyten (T-Zellen) sind Lymphozyten zu verstehen, die z. B. bei der Zellvermittelten Zytotoxizität, bei der Allergie vom verzögerten Typ und bei der Aktivierung von B-Lymphozyten beteiligt sind. Es gibt eine Vielzahl von verschiedenen Typen (Subtypen) von T-Zellen, die jeweils durch ihre Funktion und/oder ihre Zelloberflächenantigene unterschieden werden können (siehe z. B. Imm. Rev. (1993), 74 und (1992), 82; Advances in Immunology 58 (1995) 87). Derartige Oberflächenantigene werden beispielsweise als CD (Cluster of Differentiation)-Antigene bezeichnet. Typisch für alle T-Zellen ist die Expression des Antigenerkennenden T-Zell-Rezeptors. T-Zellen entwickeln sich aus hämatopoetischen Stammzellen und reifen, mit einigen Ausnahmen, im Thymus. Beispiele für T-Zellen sind zytotoxische T-Zellen, Helfer-T-Zellen, Suppressor-T-Zellen, Suppressor-inducer T-Zellen und Killer-T-Zellen.

Natural-killer-cells (NK-Zellen) sind lymphoide Zellen, die sich aus hämatopoetischen Stammzellen entwickeln und sich von T- und B-Zellen dadurch unterscheiden, daß sie weder den T-noch den B-Zell-Rezeptor exprimieren und CD3- sind.

Die Kultivierung der Lymphozyten im erfindungsgemäßen Verfahren wird in einem üblichen Basiskulturmedium durchgeführt, welches zusätzlich einen Lymphozyten-Wachstumsfaktor und Cyclosporin und/oder Ascomycin enthält. Als Basiskulturmedien sind alle Medien geeignet, die üblicherweise in der Kultur von Säugerzellen verwendet werden. Solche Kulturmedien können entweder serumhaltig oder serumfrei sein und sind jedem Fachmann bekannt. Beispiele sind RPMI 1640-Medium, Dulbecco's Modified Eagles Medium (DMEM), F12 Medium oder eine Mischung aus letzteren (DF Medium), die sowohl in serumhaltiger als auch serumfreier Form angewendet werden können. Falls serumfreie Medien verwendet werden, muß das Medium durch kritische Komponenten supplementiert werden. Solche kritischen Komponenten sind, wie jedem Fachmann bekannt, Albumin, Transferrin, Selenit und Insulin. Besonders geeignet sind auch serumfreie Medien, die alle kritischen Supplemente bereits enthalten, wie z. B. das Kulturmedium X-Vivo 20® (Bio-Whittaker, Serva).

Die Mengen an Lymphozyten-Wachstumsfaktor und an Cyclosporin und/oder Ascomycin , die im erfindungsgemäßen Verfahren zugesetzt werden, können variieren und sind im gewissen Umfang vom verwendeten Medium (serumfrei oder serumhaltig) und von der zu kultivierenden Zelle abhängig. Es hat sich gezeigt, daß bei serumfreier Kultur Mengen von 0,1 - 10 x 10⁻ ⁹ mol/l Wachstumsfaktor und 10⁻¹⁰ - 10⁻² mol/l Cyclosporin und/oder Ascomycin geeignet sind. Bei der Kultivierung von T-Zellen und/oder NK-Zellen in serumfreiem Medium ist es demnach vorteilhaft, beispielsweise 10 - 20 ng/ml IL-2 oder IL-15 und 5 - 20 ng/ml Cyclosporin-A und/oder 1 - 10 ng/ml Ascomycin zuzusetzen.

Das erfindungsgemäße Verfahren ist insbesondere zur Kultivierung und Vermehrung von Killer-T-Zellen (KT-Zellen) und tumoriziden Killer-T-Zellen geeignet. Derartige tumorizide Killer-T-Zellen können beispielsweise gemäß WO 94/23014 durch Cokultivierung von Lymphozyten, die z. B. aus Blut isoliert werden, mit Stimulatorzellen erzeugt werden. Dabei werden ruhende T-Lymphozyten zu Effektorzellen aktiviert, die Tumorzellen erkennen und abtöten oder im Wachstum inhibieren. Die aktivierten T-Lymphozyten werden in dieser Cokultivierung zur Proliferation angeregt und können durch das erfindungsgemäße Verfahren weiter kultiviert und vermehrt werden.

Ebenfalls vorteilhaft ist das erfindungsgemäße Verfahren zur Herstellung und Vermehrung von pancytotoxischen T-Zellen. Hierfür ist die Kombination eines Lymphozytenwachstumsfaktors mit Aurintricarbonsäure, oder mit einer Substanz, welche an Cyclophilin bindet und in diesem Komplex Calcineurin inhibiert, geeignet. Dazu können Blutlymphozyten nach Behandlung mit Leucyl-Leucin-Methylester durch das erfindungsgemäße Verfahren in langanhaltende Proliferation gebracht werden. Die Phenotypisierung solcher erfindungsgemäß hergestellten Zellen anhand von Oberflächenmarkern zeigt, daß die proliferierenden Zellen einheitlich T-Lymphozyten sind (100 % CD3+). Diese Zellen zeigen eine bisher noch nicht bekannte Aktivität, die im weiteren als pancytotoxische Aktivität bezeichnet wird. Pancytotoxische T-Zellen sind dadurch charakterisiert, daß sie unterschiedslos normale Zellen wie z. B. Fibroblasten, Keratinozyten oder Endothelzellen und auch Tumorzellen wie z. B. malignes Melanom, T-Lymphom oder Lungenkarzinom abtöten Pancytotoxische T-Zellen können vorteilhaft zur lokalen Behandlung von Tumoren (z. B. Tumormetastasen) eingesetzt werden.

Es hat sich überraschenderweise gezeigt, daß dann, wenn mononukleäre Blutzellen (PBMNC) ohne Vorbehandlung mit Leucyl-Leucin-Methylester kultiviert werden,. üblicherweise nach einer Latenzzeit von 14 - 28 Tagen Lymphozyten auswachsen, die sich beliebig vermehren lassen. Die Phenotypisierung dieser Zellen zeigt, daß sie ein Gemisch aus T-Zellen (CD2+, CD3+) und NK-Zellen (CD2+, CD3-, CD16+) darstellen. Funktionell sind sie ebenfalls pancytotoxisch und somit von KT-Zellen unterschiedlich. Auch hier ist es bevorzugt, als Lymphozytenwachstumsfaktor IL2 oder IL15 und Cyclosporin oder Ascomycin zu verwenden.

Die folgenden Beispiele, Publikationen und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.
- **Fig. 1**: zeigt das Wachstumsverhalten von Killer-T-Zellen in serumfreiem DF-Medium bei Zugabe von IL-2 + CsA (A) und CsA allein (B).
- **Fig. 2**: zeigt das Wachstumsverhalten von Killer-T-Zellen in serumfreiem DF-Medium bei Zugabe von IL-15 und CsA (A) und IL-15 allein (B).
- **Fig. 3**: zeigt das Wachstumsverhalten von Killer-T-Zellen in serumfreiem DF-Medium bei Zugabe von IL-2 und Ascomycin (FK 520) (A) und Ascomycin allein (B).
- **Fig. 4**: zeigt das Wachstumsverhalten von Killer-T-Zellen in serumfreiem DF-Medium bei Zugabe von IL-2 und Aurintricarbonsäure (A) und Arintricarbonsäure allein (B).

### Beispiel 1

### Herstellen von Killer-T-Zellen

Mononukleäre Zellen aus dem peripheren Blut (PBMNC) menschlicher Spender werden mittels Gradientenzentrifugation (Lymphozytentrennmedium, BM) isoliert, zweimal mit Phosphat-gepufferter Salzlösung gewaschen und in einer Dichte von 5 - 10 x 10⁶ Zellen/ml DF-Medium nach Thiele und Lipsky (J. Immunol. 136 (1986) 1038-1048) mit 250 µM Leucyl-Leucin-Methylester (BM) über 20 Minuten bei Raumtemperatur inkubiert. Nach Waschen mit DF-Medium werden die Zellen in einer Dichte von 1 - 2 x 10⁶ pro ml DF-Medium zusammen mit bestrahlten (2000 rad) HB654- oder HB617-Zellen (Stimulatorzellen; 2 - 5 x 10⁵ pro ml) bei 37°C in 8 % CO₂-Atmosphäre kultiviert. Am Tag 5 - 6 der Kokultur wird die Hälfte bis Zweidrittel des Kulturmediums erneuert und nochmals bestrahlte Stimulatorzellen (2 - 5 x 10⁵ pro ml) zugegeben. Ab Tag 8 - 10 nach Beginn der Kokultur, wenn alle Stimulatorzellen durch die zytotoxische Aktivität der Killer-T-Zellen zerstört worden sind, werden die Killer-T-Zellen für die folgenden Beispiele verwendet.

Die Phänotypisierung der Zellen am Tag 10 der Kokultur ergibt, daß >95 % der Zellen CD3⁺, ca. 40 % CD4⁺ und ca. 60 % CD8⁺ sind. Zellen mit den Markern CD19 oder CD16 werden nicht gefunden.

### Beispiel 2

### Vermehrung von Killer-T-Zellen in serumfreiem Medium, das Interleukin-2 (IL-2) und Cyclosporin A (CsA) enthält.

Killer-T-Zellen, die gemäß Beispiel 1 hergestellt worden sind, werden einmal in DF-Medium gewaschen und in einer Dichte von 5 x 10⁵ pro ml DF-Medium in zwei getrennten Ansätzen, die mit A und B gekennzeichnet werden, kultiviert. Dem Ansatz A werden humanes, rekombinantes IL-2 (BM; 20 ng/ml) und CsA (Sandoz; 12,5 ng/ml) zugesetzt. Zu Ansatz B wird nur CsA (12,5 ng/ml) gegeben. Jeden zweiten Tag wird die Hälfte des Kulturmediums erneuert, und die Zellzahl auf 5 x 10⁵ pro ml eingestellt.

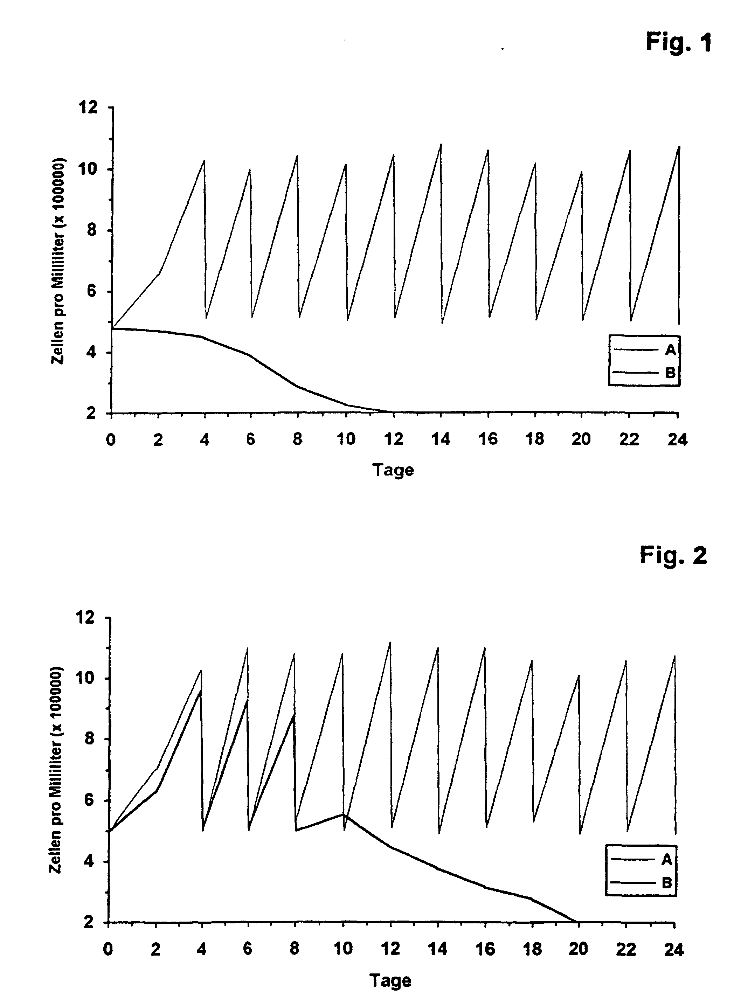

Wie in Figur 1 dargestellt, vermehren sich die Killer-T-Zellen im Ansatz A (IL-2 + CsA) mit einer Verdopplungszeit von etwa 48 Stunden über mindestens 10 Verdopplungszyklen (entspricht einer 1000-fachen Vermehrung). Im Ansatz B (nur CsA) vermehren sich die Killer-T-Zellen nicht.

### Beispiel 3

### Vermehrung von Killer-T-Zellen in serumfreiem Medium, das Interleukin-15 (IL-15) und Cyclosporin A (CsA) enthält.

Killer-T-Zellen, die gemäß Beispiel 1 hergestellt worden sind, werden einmal in DF-Medium gewaschen und in einer Dichte von 5 x 10⁵ pro ml DF-Medium in zwei getrennten Ansätzen, die mit A und B gekennzeichnet werden, kultiviert. Dem Ansatz A werden humanes, rekombinantes IL-15 (R & D Systems; 15 ng/ml) und CsA (Sandoz; 12,5 ng/ml) zugesetzt. Zu Ansatz B wird nur IL-15 (15 ng/ml) gegeben. Jeden zweiten Tag wird die Hälfte des Kulturmediums erneuert und die Zellzahl auf 5 x 10⁵ pro ml eingestellt.

Wie in Figur 2 dargestellt, vermehren sich die Killer-T-Zellen im Ansatz A (IL-15 + CsA) mit einer Verdopplungszeit von etwa 48 Stunden über mindestens 11 Verdopplungszyklen. Im Ansatz B (nur IL-15) stagniert die Proliferation nach 3 Verdopplungszyklen.

### Beispiel 4

### Vermehrung von Killer-T-Zellen in serumfreiem Medium, das Interleukin-2 (IL-2) und Ascomycin (FK 520) enthält.

Killer-T-Zellen, die gemäß Beispiel 1 hergestellt worden sind, werden einmal in DF-Medium gewaschen und in einer Dichte von 5 x 10⁵ pro ml DF-Medium in zwei getrennten Ansätzen, die mit A und B gekennzeichnet werden, kultiviert. Dem Ansatz A werden rh IL-2 (20 ng /ml) und Ascomycin (Calbiochem; 2,5 ng/ml) zugesetzt. Zu Ansatz B wird nur Ascomycin (2,5 ng/ml) gegeben. Jeden zweiten Tag wird die Hälfte des Kulturmediums erneuert und die Zellzahl auf 5 x 10⁵ pro ml eingestellt.

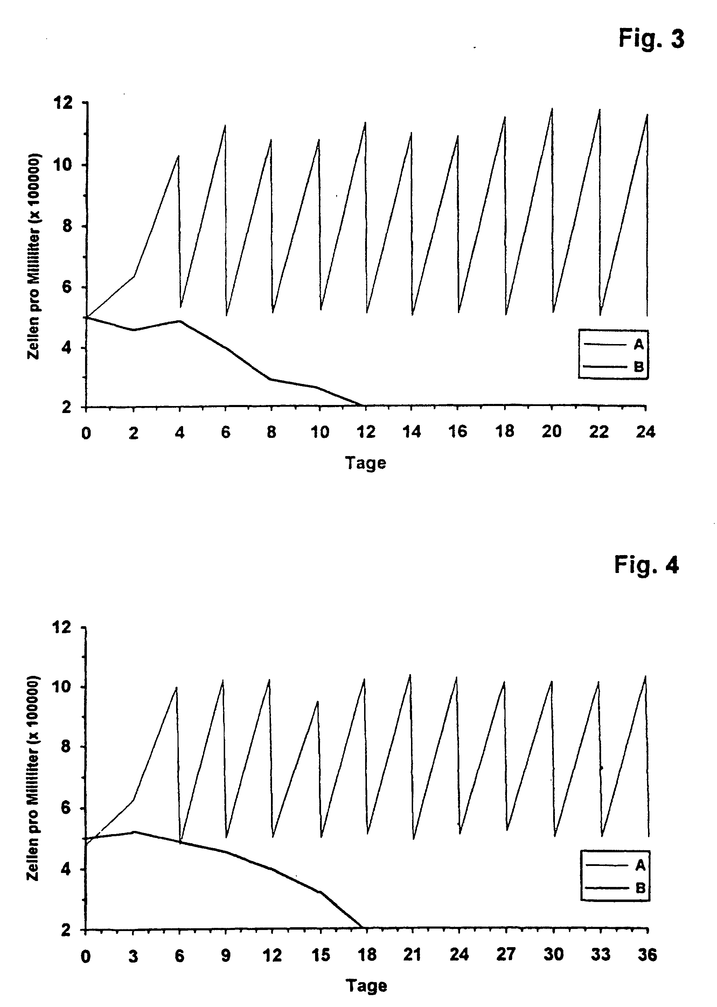

Wie in Figur 3 dargestellt, vermehren sich die Killer-T-Zellen im Ansatz A (IL-2 + Ascomycin) mit einer Verdopplungszeit von etwa 48 Stunden über mindestens 10 Verdopplungszyklen. In Ansatz B (nur Ascomycin) wird keine Vermehrung der Killer-T-Zellen beobachtet.

### Beispiel 5

### Vermehrung von NK- und T-Lymphozyten aus mononukleären Zellen des menschlichen Blutes in serumfreiem Medium, das Interleukin-2 (IL-2) und Transforming-Growth-Factor-β₁ (TGF-β1) enthält.

Mononukleäre Zellen aus dem peripheren Blut (PBMNC) eines menschlichen Spenders werden mittels Gradientenzentrifugation (Lymphozytentrennmedium, Boehringer Mannheim GmbH, Deutschland (BM)) isoliert, zweimal mit Phosphat-gepufferter Salzlösung gewaschen und in einer Dichte von 1 x 10⁶ Zellen/ml in DF-Medium aufgenommen und in drei getrennten Ansätzen, die mit A, B und C gekennzeichnet werden, kultiviert. Zu Ansatz A werden rekombinantes, humanes IL-2 (BM, 20 ng/ml) und rh TGF-β₁ (BM, 4 ng/ml) gegeben, zu Ansatz B wird nur IL-2 (20 ng/ml) und zu Ansatz C nur TGF-β₁ (4 ng/ml) gegeben. Jeden zweiten Tag wird die Hälfte des Kulturmediums (+Zytokin[en]) erneuert und die Zellzahl in den ersten 7 Tagen auf ca. 1 x 10⁶ Zellen/ml, danach auf ca. 0,5 x 10⁵ Zellen/ml eingestellt.

In Ansatz A (IL-2 + TGF-β1) vermehrt sich die Zahl der nichtadhärenten, lymphoiden Zellen etwa ab Tag 5 nach Beginn der Kultur, zunächst mit einer Verdopplungszeit von ca. 96 Stunden, ab Tag 20, wenn keine Kolonien mit adhärenten, monozytären Zellen mehr erkennbar sind, mit einer Verdopplungszeit von weniger als 48 Stunden. Am Tag 50 der kontinuierlichen Kultivierung hat sich die Zellzahl, gemessen an der Eingabe, um das mehr als 10⁴-fache vergrößert. Die Analyse der Zellen anhand ihrer Oberflächenmarker ergibt am Tag 50 folgendes Ergebnis: Die Population enthält
* ca. 50% NK-Zellen (CD2+, CD3-, CD16+, CD56+), und
* ca. 50% T-Zellen (CD3+, CD4+, CD8+)

Im Ansatz B (nur IL-2) vermehren sich lymphoide Zellen über ca. 16 Tage nur mäßig (<50fach), stagnieren sodann und sterben nach ca. 20 weiteren Tagen ab.

Im Ansatz B (nur TGF-β₁) vermehren sich die Zellen nicht.

### Beispiel 6

### Vermehrung von Killer-T-Zellen in serumfreiem Medium, das Interleukin 2 (IL-2) und Aurintricarbonsäure (ATA) enthält.

Killer-T-Zellen, die gemäß Beispiel 1 hergestellt worden sind, werden einmal mit DF-Medium gewaschen und in einer Dichte von 5 x 10⁵ pro ml DF-Medium in zwei getrennten Ansätzen, die mit A und B gekennzeichnet werden, kultiviert. Dem Ansatz A werden IL-2 (20 ng/ml) und Aurintricarbonsäure (Aldrich Chemie; 4,2 µg/ml) zugesetzt. Zu Ansatz B wird nur Aurintricarbonsäure (4,2 µg/ml) gegeben. Jeden dritten Tag wird die Hälfte des Kulturmediums erneuert und die Zellzahl auf 5 x 10⁵ pro ml eingestellt.

Wie in Figur 4 dargestellt, vermehren sich die Killer-T-Zellen im Ansatz A (IL-2 + ATA) mit einer Verdoppelungszeit von etwa 72 Stunden über mindestens 11 Verdoppelungszyklen. Im Ansatz B (nur ATA) vermehren sich die Killer-T-Zellen nicht.

### Beispiel 7

### Vermehrung von Tumor-infiltrierenden Lymphozyten in einem Medium, das Interleukin-2 (IL-2) und Aurintricarbonsäure (ATA) enthält.

Der Knoten eines menschlichen Kolonkarzinoms, der durch Operation aus dem Dickdarm entfernt wurde, wird von Bindegewebe und normalen Darmanteilen befreit und in ca. 2 x 2 x 3 mm große Teile zerschnitten. Die Tumorfragmente werden in Iscove-modifiziertem DME-Medium (Gibco), das 15 % FKS (BM) enthält, aufgenommen und gleichmäßig auf vier Kulturschälchen, die mit A, B, C und D bezeichnet werden, verteilt. Dem Ansatz A wird IL-2 (20 ng/ml), dem Ansatz B Aurintricarbonsäure (4,2 µg/ml) und dem Ansatz C IL-2 (20 ng/ml plus Aurintricarbonsäure (4,2 µg/ml) zugesetzt. Der Ansatz D bleibt ohne Zusätze. Jeden zweiten Tag wird Dreiviertel der jeweiligen Kulturmedien ohne Veränderung des Gewebeund Zellinhaltes erneuert. Die mikroskopische Kontrolle der Kulturen ergibt, daß aus den Tumorgewebestücken nach 24 - 48 Stunden lymphozytäre Zellen auswandern, die sich in der Teilkultur C, die IL-2 plus ATA enthält, nicht aber in den Teilkulturen A, B oder D vermehren. Nach zehntägiger Kultur ergibt sich folgendes Bild: In A und B (nur IL-2 oder ATA) ist die Zahl der ausgewanderten Lymphozyten im Vergleich zu Tag 2 konstant geblieben, in D (keine Zusätze) sind kaum noch Lymphozyten nachweisbar. In der Teilkultur C hat sich die Zahl der Lymphozyten im Vergleich zu A oder B auf das 20-fache erhöht. Die Lymphozyten aus C werden aus der Teilkultur C isoliert und anhand ihrer Oberflächenmarker analysiert. Die Population enthält
ca. 60 % T-Lymphozyten (CD3+, CD4+, CD8+, CD19-)
ca. 25 % NK-Zellen (CD2+, CD3-, CD56+, CD19-) und
ca. 15 % B-Lymphozyten (CD19+, CD3-).

### Referenzliste

Advances in Immunology 58 (1995) 87
Ayoub, I.A., Immunological Invest. 25 (1996) 129 - 151
Baughman-G., et al., Mol-Cell-Biol. 15 (1995) 4395 - 402
Callard, RE., und Gearing, J.H., The Cytokine Facts Book, Academic Press, London 1994
Cerwenka, A. J. Immunol. 156 (1996) 459 - 464
EP-A 0 203 403
Imm. Rev. (1992) 82
Imm. Rev. (1993) 74
Inne, T.H.,et al., J. Immunol. 148 (1992) 3847 - 3856
Jong de, R., Intern. Immunol. 6 (1994) 631 - 638
Kakalis, LT., et al., FEBS-Lett. 362 (1995) 55 - 8
Kawamura, A., et al., J-Biol-Chem. 270 (1995) 15463 - 6
Kroemer, G., Advances in Immunology 58 (1995) 211-296
Kutnik, V., et al., Period. Biol. 92 (1990) 48
Pazderka-F., et al., Transpl-Immunol. (1996) 23 - 31
Pierson, B.A. et al., Blood 87 (1996) 180 - 189
Rusnak-F., et al., Bone-Marrow-Transplant. 17 (1996) 309 - 13
Su-Q., et al., Ren-Physiol-Biochem. 18 (1995) 128 - 39
Thiele und Lipsky, J. Immunol. 136 (1986) 1038-1048
WO90/10059
WO94/23014
Gonzales, R.G. et al.: Biochemistry 19: 4299 - 4303 (1980)

## Patentansprüche

1. Verfahren zur Vermehrung von Lymphozyten in einer Zellkultur, **dadurch gekennzeichnet, dass** Lymphozyten in einem Zellkulturmedium kultiviert und vermehrt werden, welches einen Lymphozytenwachstumsfaktor sowie zusätzlich Aurintricarbonsäure oder eine Substanz, welche an ein Cyclophilin bindet und in diesem Komplex Calcineurin inhibiert, enthält.

2. Verfahren nach Anspruch 1, wobei das Zellkulturmedium als cyclophilinbindende Substanz Cyclosporin, Ascomycin und/oder Tacrolimus enthält.

3. Verfahren nach Anspruch 1 oder 2, wobei die Lymphozyten Leukozyten sind, die im Blut, in der Lymphe, in der Milz, in Lymphknoten, in Tumoren, oder in entzündeten Geweben zu finden sind.

4. Verfahren nach einem der Ansprüche 1 bis 3, wobei die Lymphozyten T-Lymphozyten, B-Lymphozyten oder NK-Zellen sind.

5. Verfahren nach einem der Ansprüche 1 bis 4, wobei der Lymphozytenwachstumsfaktor ein T-Lymphozyten-, ein B-Lymphozyten- oder ein NK-Lymphozyten-Wachstumsfaktor ist und vorzugsweise aus der Gruppe IL-2, IL-10, IL-13, IL-14 und IL-15 ausgewählt ist.

6. Verfahren nach einem der Ansprüche 1 bis 6, **dadurch gekennzeichnet, dass** als Lymphozyten tumorinfiltrierende Lymphozyten kultiviert werden und nach zehntägiger Kultur ein Gemisch aus T-Lymphozyten (CD3+, CD4+, CD8+, CD19-), NK-Zellen (CD2+, CD3-, CD56+, CD19-) und B-Lymphozyten (CD19+, CD3-) nachweisbar ist.

7. Verfahren nach einem der Ansprüche 1 bis 6, **dadurch gekennzeichnet, dass** als Lymphozyten Blutlymphozyten, nach Behandlung mit Leucyl-Leucin-Methylester, eingesetzt werden, wobei T-Lymphozyten proliferieren, die sowohl normale Zellen als auch Tumorzellen abtöten können.

## Claims

1. Process for multiplying lymphocytes in a cell culture, **characterized in that** lymphocytes are cultivated and multiplied in a cell culture medium which contains a lymphocyte growth factor and additionally aurintricarboxylic acid or a substance which binds to a cyclophilin and, in this complex, inhibits calcineurin.

2. Process according to Claim 1, the cell culture medium containing cyclosporin, ascomycin and/or tacrolimus as the cyclophilin-binding substance.

3. Process according to Claim 1 or 2, the lymphocytes being leukocytes which are to be found in the blood, in the lymph, in the spleen, in lymph nodes, in tumours or in inflamed tissues.

4. Process according to any of Claims 1 to 3, the lymphocytes being T-lymphocytes, B-lymphocytes or NK cells.

5. Process according to any of Claims 1 to 4, the lymphocyte growth factor being aT-lymphocyte, a B-lymphocyte or a NK lymphocyte growth factor and preferably being selected from the group IL-2, IL-10, IL-13, IL-14 and IL-15.

6. Process according to any of Claims 1 to 6, **characterized in that** the lymphocytes cultivated are tumour-infiltrating lymphocytes and, after culture for ten days, a mixture of T-lymphocytes (CD3+, CD4+, CD8+, CD19-), NK cells (CD2+, CD3-, CD56+, CD19-) and B-lymphocytes (CD19+, CD3-) is detectable.

7. Process according to any of Claims 1 to 6, **characterized in that** the lymphocytes used are blood lymphocytes, after treatment with leucyl-leucin methyl ester, T-lymphocytes, which can kill both normal cells and tumour cells, proliferating.

## Revendications

1. Procédé pour multiplier des lymphocytes en culture cellulaire, **caractérisé en ce que** les lymphocytes sont cultivés, pour se multiplier, dans un milieu pour culture cellulaire, lequel contient un facteur de croissance des lymphocytes, ainsi qu'en plus, de l'acide aurine-tricarboxylique ou une substance qui se lie à une cyclophiline et qui inhibe, dans ce complexe, la calcineurine.

2. Procédé selon la revendication 1, dans lequel le milieu de culture contient, en tant que substance se liant à une cyclophiline, la cyclosporine, l'ascomycine et / ou le tacrolimus.

3. Procédé selon la revendication 1 ou la revendication 2, où les lymphocytes sont des leucocytes se trouvant dans le sang, dans la lymphe, la rate, les ganglions lymphatiques, les tumeurs ou dans des tissus enflammés.

4. Procédé selon l'une des revendications 1 à 3, où les lymphocytes sont des lymphocytes T, des lymphocytes B ou des cellules NK.

5. Procédé selon l'une des revendications 1 à 4, où le facteur de croissance des lymphocytes est un facteur de croissance des lymphocytes T, des lymphocytes B ou des lymphocytes NK, ce facteur étant choisi, de préférence, dans le groupe constitué par IL-2, IL-10, IL-13, IL-14 et IL-15.

6. Procédé selon l'une des revendications 1 à 5, **caractérisé en ce que** les lymphocytes cultivés sont des lymphocytes ayant infiltré une tumeur et **en ce qu'**après dix jours de culture, on obtient, de manière démontrable, un mélange de lymphocytes T (CD3+, CD4+, CD8+, CD19 -), de cellules NK (CD2+, CD3-, CD56+, CD19-) et de lymphocytes B (CD19+, CD3-).

7. Procédé selon l'une des revendications 1 à 6, **caractérisé en ce que** les lymphocytes utilisés sont des lymphocytes du sang, obtenus après un traitement par l'ester méthylique de la leucyl-leucine et où les lymphocytes T, qui prolifèrent, sont capables de tuer, aussi bien les cellules normales, que les cellules tumorales.
